(19) Europäisches Patentamt
European Patent Office
Office européen des brevets

(11) Veröffentlichungsnummer: **0 080 718**
**B1**

(12) # EUROPÄISCHE PATENTSCHRIFT


(45) Veröffentlichungstag der Patentschrift: **14.05.86**

(21) Anmeldenummer: **82110986.5**

(22) Anmeldetag: **27.11.82**

(51) Int. Cl.[4]: **C 07 D 307/935**, C 07 C 177/00, C 07 C 69/76, C 07 C 65/00, A 61 K 31/34, A 61 K 31/557, A 61 K 31/19, A 61 K 31/235


(54) **Inter-m-Phenylen-PGI2-Derivate, Verfahren zu deren Herstellung und diese Verbindungen enthaltende pharmazeutische Präparate.**


(30) Priorität: **01.12.81 HU 360081**
**22.10.82 HU 337882**

(43) Veröffentlichungstag der Anmeldung: **08.06.83 Patentblatt 83/23**

(45) Bekanntmachung des Hinweises auf die Patenterteilung: **14.05.86 Patentblatt 86/20**

(84) Benannte Vertragsstaaten: **CH DE FR GB IT LI**

(56) Entgegenhaltungen:
**EP - A - 0 045 842**
**EP - A - 0 062 902**
**GB - A - 2 013 661**
**GB - A - 2 070 596**



(73) Patentinhaber: **CHINOIN Gyogyszer és Vegyészeti Termékek Gyára RT., To utca 1-5, H-1045 Budapest IV (HU)**

(72) Erfinder: **Székely, István, Dr., Dipl.-Chem., Krajcár u. 6, H-2120 Dunakeszi (HU)**

(72) Erfinder: **Botár, Sándor, Dipl.-Chem., Baross u. 109, H-1082 Budapest (HU)**
Erfinder: **Lovász Gáspár, Marianna, Dr., Dipl.-Ing. Chem., Lakatos u. 28, H-1184 Budapest (HU)**
Erfinder: **Dolgos Kékesi, Krisztina, Aprilis 4 u. 23, H-4029 Debrecen (HU)**
Erfinder: **Kovács, Gábor, Dr., Dipl.-Ing. Chem., Róna park 4, H-1142 Budapest (HU)**
Erfinder: **Virág, Sándor, Dr., Sallai I. u. 29/a, H 1136 Budapest (HU)**
Erfinder: **Szücs, Tamás, Dr., Hegedüs Gy u. 7, H-1136 Budapest (HU)**
Erfinder: **Rákoczy, István, Dr., Gombócz Z. u. 16, H-1118 Budapest (HU)**
Erfinder: **Tihanyi, Károly, Dr., Pestiu. 161, H-1173, Budapest (HU)**
Erfinder: **Körmöczy, Péter, Dr., Uri u. 33, H-1014 Budapest (HU)**
Erfinder: **Hadházy, Pál, Dr., Baranyai u. 31/b, H-1117 Budapest (HU)**
Erfinder: **Stadler, István, Dr., Népstadion u. 18, H-1143 Budapest (HU)**
Erfinder: **Blaskó, György, Dr., Pozsonyi u. 4/a, H-1045 Budapest (HU)**
Erfinder: **Kószegi, Béla, Dipl.-Chem., Corvin krt. 52, H-1192 Budapest (HU)**

(74) Vertreter: **Lotterhos, Hans Walter, Dr.-Ing., Lichtensteinstrasse 3, D-6000 Frankfurt am Main 1 (DE)**


EP 0 080 718 B1


Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue inter-m-Phenylenprostacyclin-Analoge der allgemeinen Formel I

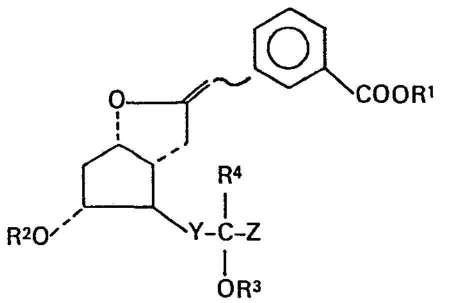

(I)

worin

$R^1$ Wasserstoff, eine $C_{1-4}$-Alkylgruppe, ein physiologisch verträgliches primäres, sekundäres, tertiäres oder quaternäres Ammoniumkation oder ein Metallkation,

$R^2$ und $R^3$ Wasserstoff,

$R^4$ Wasserstoff oder eine $C_{1-4}$-Alkylgruppe,

Y -C≡C- oder die Gruppierung trans-CH=CW- bedeuten, in der W = Chlor, Brom oder Fluor ist und

Z eine gegebenenfalls durch $C_{1-4}$-Alkyl oder Fluor ein- oder mehrfach substituierte $C_{6-9}$-Alkylgruppe oder gegebenenfalls durch Phenyl, $C_{1-4}$-Alkyl, $C_{1-4}$-Alkoxy, Halogen, Trifluormethyl oder Nitro substituierte Phenylmethyl- oder Naphthylmethylgruppe oder gegebenenfalls durch Phenyl, $C_{1-4}$-Alkyl, $C_{1-4}$-Alkoxy, Halogen, Trifluormethyl oder Nitro substituierte Phenoxymethyl- oder Naphthoxymethylgruppe bedeutet.

Die Erfindung betrifft ferner Arzneimittelpräparate, die diese Verbindungen enthalten und hauptsächlich eine antiaggregative Wirkung aufweisen.

Die neuen Verbindungen der Erfindung haben eine Antiaggregationswirkung, die der von $PGI_2$ vergleichbar ist, jedoch eine hypotensive Wirkung, die um Grössenordnungen geringer als die des $PGI_2$ ist, d.h. ihre Wirkungsselektivität ist um Grössenordnungen besser als die des $PGI_2$. Ferner sind die neuen Verbindungen stabiler als $PGI_2$. Aus diesen Gründen können die Verbindungen in der Humanmedizin und auf veterinärmedizinischem Gebiet zur Hemmung und Beeinflussung der Blutgerinnung sowie zum Auflösen bereits vorhandener Thromben therapeutisch verwendet werden.

Das Prostacyclin ($PGI_2$) wurde 1976 entdeckt (Nature, 1976, 263 und 663; Prostaglandins 1976, 12, 915; Angew. Chem. Int. Ed. Engl. 1978, 17, 293).

Es wurde festgestellt, dass das Prostacyclin das stärkste bekannte Antiaggregations- und Desaggregationsmittel ist, das ausserdem noch weitere pharmazeutische Wirkungen aufweist, z.B. lässt es die Herzkranzgefässe erschlaffen und erweitert die Adern des peripheren Blutkreislaufs. Seit der Entdeckung des Prostacyclins sind intensive Untersuchungen gemacht worden mit dem Ziel, Verbindungen zu synthetisieren, deren anti- und desaggregierende Wirkung der des Prostacyclins nahekommt, die jedoch chemisch stabiler und in der Wirkung selektiver sind als die Natursubstanz Prostacyclin, und die in Richtung der bekannten Metabolismuswege einen gehemmten Metabolismus aufweisen.

In der DE-OS 2 945 781 sind Prostacyclin-Analoge beschrieben, die in der Seitenkette mit der Carboxylgruppe eine o-Phenylengruppe aufweisen. Für diese Verbindungen ist ein sehr breites Wirkungsspektrum angegeben. In einer Dosis von 0,01–50 µg/kg·min i.v. sollen sie die glatte Muskulatur stimulieren, in Dosen von 0,01–10 mg/kg i.v. die Bluttplättchenaggregation beeinflussen, in Dosen von 0,01–50 µg/kg·min i.v. den Blutdruck senken, in Dosen von 0,1–20 µg/kg·min i.v. die Magensäuresekretion herabsetzen und in nicht näher bezeichneten Dosen sollen sie zur Behandlung von Magen-Darm-Beschwerden, die durch nicht-steroide entzündungshemmende Stoffe ausgelöst worden sind, geeignet sein können. Ausserdem wird für Dosen von 0,01–5 mg/kg p.o. eine die Bronchien erweiterende Wirkung (gegen Asthma) und für nicht definierte Dosen eine Wirkung gegen unterschiedliche Hautkrankheiten angegeben. Diese Aufzählung zeigt, dass die in der DE-OS beschriebenen Verbindungen über viele Wirkungen gleichzetig verfügen. Jedoch ausser der gewünschten Hauptwirkung sind alle weiteren Wirkungen als unerwünschte Nebenwirkungen anzusehen, die therapeutisch nachteilig sind, weil sie bei der Behandlung immer neben der angestrebten Hauptwirkung auftreten und weil ihre Unterdrückung die Verabreichung weiterer Arzneimittel erfordert.

In der EP-A1-0 045 842 sind $PGI_2$-Analoge beschrieben, die in der Seitenkette mit der Carboxylgruppe eine m-Phenylengruppe enthalten. Diese Verbindungen zeigen im Vergleich zu den 5,6-Dihydroprostacyclinen eine geringere Wirkungsvielfalt und insbesondere bezüglich der unerwünschten Blutdrucksenkung eine bessere Selektivität. Ausserdem sind sie stabiler als Prostacyclin. Die in der EP-A1-0 045 842 beschriebenen Verbindungen enthalten in 13,14-Stellung eine Äthylen-, Vinylen- oder –C≡C-Gruppe und an Stelle der 16,20-Alkylgruppe eine in 1-Stellung gegebenenfalls mono- oder disubstituierte Pentylgruppe oder eine in 4-Stellung gegebenenfalls substituierte Cyclohexylgruppe. Die Verbindungen können ausserdem in 15-Stellung gegebenenfalls durch eine Methylgruppe substituiert sein.

Im Prinzip von ähnlicher Struktur sind die in der EP-A1-0 062 902 beschriebenen Verbindungen, die sich vom $PGI_2$ dadurch unterscheiden, dass sie in der Seitenkette mit der Carboxylgruppe eine m-Phenylgruppe, in der unteren Seitenkette in 20-Stellung noch eine Methyl- oder Äthylgruppe oder statt der Seitenkette in 16,20-Stellung eine Phenoxymethyl-, m-Trifluormethyl-phenoxymethyl- oder eine in der Aminogruppe gegebenenfalls substituierte 1-Aminoalkylgruppe mit 4–6 Kohlenstoffatomen oder eine in der Aminogruppe gegebenenfalls substituierte Aminophenyl- bzw. Aminobenzylgruppe enthalten. Die Antiaggregationswirkung dieser Verbindungen ist zwar um etwa eine Grössenordnung geringer als die von $PGI_2$, jedoch ihre blutdrucksenkende Wirkung beträgt nur ein Fünfzigstel bis ein Achtzigstel des $PGI_2$ und in der Stabilität übertreffen sie das $PGI_2$ bei weitem.

Es wurde überraschender Weise gefunden, dass bei den Verbindungen der Erfindung die antiaggregative Wirkung noch gesteigert werden konnte, während gleichzeitig die blutdrucksenkende Wirkung noch weiter erniedrigt wurde, aber die Stabilität erhalten blieb.

In einem Vergleichsversuch wurde die antiaggregative und die blutdrucksenkende Wirkung der folgenden Verbindungen miteinander vergleichen:

$PGI_2$,
5(Z)-2,3,4-Trinor-1,5-inter-m-phenylen-$PGI_2$ (EP-A1-0 045 842/A),
5(Z)-2,3,4-Trinor-1,5-inter-m-phenylen-13,14-didehydro-$PGI_2$ (EP-A1-0 045 842/B),
5(Z)-2,3,4-Trinor-1,5-inter-m-phenylen-20-methyl-$PGI_2$ (EP-A1-0 062 902),
5(Z)-2,3,4-Trinor-1,5-inter-m-phenylen-13,14-didehydro-20-methyl-$PGI_2$ (Verbindung A) und
5(Z)-2,3,4-Trinor-1,5-inter-m-phenylen-13,14-didehydro-20-äthyl-$PGI_2$ (Verbindung B).

Die Verbindungen wurden in Form ihrer Natriumsalze eingesetzt.

Die Thrombocytenaggregation wurde im Zweikanal-Aggregometer nach Payton bei einem Blutvolumen von 0,5 ml gemessen. Das zu den Messungen erforderliche Blut wurde Personen entnommen, die mindestens zwei Wochen vor der Blutentnahme keine die Thrombocytenaggregation beeinflussenden Arzneimittel eingenommen hatten und die weder eine Nieren- noch eine Lebererkrankung aufwiesen. Das kubitale venöse Blut wurde im Verhältnis 9:1 mit 3,8%iger Na-Citratlösung verdünnt und dann – zur Gewinnung eines thrombocytenreichen Plasmas (PRP) – 5 Minuten mit 230 g zentrifugiert, während thrombocytenarmes Plasma nach 10 Minuten Zentrifugieren erhalten wurde. Die Anzahl der Thrombocyten wurde auf einen Wert von $2–3 \times 10^5$ pro Milliliter eingestellt. Die Untersuchungen wurden – von der Blutentnahme an gerechnet – innerhalb einer Stunde vorgenommen. Vor der Messung wurde die Prostacyclin-Empfindlichkeit des jeweiligen Plasmas kontrolliert. Bei thrombocytenreichen Plasmas ergab sich für Prostacyclin ein $IC_{50}$-Wert von 0,6–1,0 ng/ml.

Die Aggregation der Thrombocyten wurde durch 2 $\mu$M ADP ausgelöst. Die untersuchten Verbindungen wurden in einem 0,1 M Tris-HCl-Puffer des pH-Wertes 7,8 gelöst und bei 0–4°C gehalten. Manche Verbindungen wurden nach einer gewissen Zeit erneut gemessen und so lange bei 0–4°C aufbewahrt. Aus den Ergebnissen wurde der jeweilige $IC_{50}$-Wert graphisch bestimmt.

Die blutdrucksenkende Wirkung wurde an narkotisierten Ratten untersucht. Aus den Versuchsergebnissen wurde die zur Senkung des Blutdrucks um 25 mmHg (3,33 kPa) erforderliche Dosis ($ED_{25}$) berechnet.

In der folgenden Tabelle sind die auf $PGI_2$ bezogenen relativen Werte der aggregationshemmenden und blutdrucksenkenden Wirkung angegeben.

Tabelle

| Untersuchte Verbindung | Antiaggregative Wirkung $IC_{50}PGI_2/IC_{50}$-Test | Blutdrucksenkende Wirkung $ED_{25}PGI_2/ED_{25}$-Test |
|---|---|---|
| $PGI_2$ | 1/1 | 1/1 |
| EP-A1-0 045 842/A | 1/50–1/70 | 1/10 |
| EP-A1-0 045 842/B | 1/20–1/30 | 1/20 –1/30 |
| EP-A1-0 062 902 | 1/15–1/20 | 1/50 –1/80 |
| Verbindung A | 1/2 –1/4 | 1/100–1/150 |
| Verbindung B | 1/1 –1/2 | 1/200–1/230 |

Aus der Tabelle ist ohne weiteres ersichtlich, dass die Verbindungen A und B – was die Wirkungsstärke der Nebenwirkungen betrifft – wesentlich vorteilhafter sind, als die bekannten Verbindungen. Insbesondere trifft das für die Verbindung B zu, deren nützliche (d.h. antiaggregative) Wirkung im wesentlichen der des $PGI_2$ entspricht, während die zu den unerwünschten Nebenwirkungen zählende blutdrucksenkende Wirkung nur etwa 1/200 der blutdrucksenkenden Wirkung des $PGI_2$ beträgt. Für die Stabilität der erfindungsgemässen Verbindungen ist charakteristisch, dass auch in stark saurem Medium die Halbwertszeit ihrer Zersetzung noch mehrere Tage beträgt; aus den Verbindungen können demnach auch zur oralen Verabreichung geeignete Präparate hergestellt werden. Die Verbindung der Erfindung haben ausser der – mit Prostacyclin verglichen – grösseren chemischen Stabilität und der starken antiaggregativen und desaggregativen Wirkung den weiteren Vorteil, dass ihre Wirkungsdauer die des Prostacyclins um ein Mehrfaches übertrifft. Der Metabolismus des natürlichen Prostacyclins verläuft sehr rasch. Durch Bestimmung der Produkte des Metabolismus auf physikalisch-chemischem Wege konnte

nachgewiesen werden, dass die Verbindung entweder durch chemische Hydrolyse oder durch β-Oxydation der oberen Kette oder durch Oxydation der 15-Hydroxylgruppe zur 15-Ketogruppe abgebaut wird.

Bei den Verbindungen der Erfindung sind diese Angriffsstellen des Metabolismus gehemmt. Der in die sog. obere Kette eingebaute aromatische Ring schliesst die aliphatische β-Oxydation von vornherein aus. Die für die Wirkung kritische Kettenlänge ändert sich auch durch andere metabolistische Reaktionen der Phenylgruppe (z.B. Hydroxylierung) nicht.

Für die Oxydation der 15-Hydroxylgruppe ist das sog. 15-Hydroxy-prostaglandin-dehydrogenase-Enzymsystem (PGDH) verantwortlich. Aus der Chemie der natürlichen Prostaglandine ist bekannt, dass strukturell in gewisser Weise modifizierte Prostaglandine nicht mehr die Substrate dieses Enzymsystems sind, d.h. dass keine Inaktivierung erfolgt.

Zur Bestimmung der Wirkungsdauer wurde die sog. «ex vivo»-Methodik gewählt, die Versuchstiere waren Kaninchen. Die Wirkungsdauer der Verbindungen war hinsichtlich der Antiaggregation 5–10mal so lang wie die des Prostacyclins.

Die Erfindung betrifft auch die Herstellung der Verbindungen der allgemeinen Formel I, die dadurch gekennzeichnet ist, dass man Lactole der allgemeinen Formel II

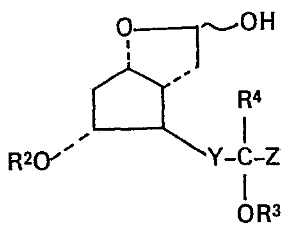

(II)

worin
$R^4$, Y und Z die oben angegebene Bedeutung haben und
$R^2$ und $R^3$ unabhängig voneinander Wasserstoff, eine $C_{1-6}$-Alkanoyl-, gegebenenfalls durch Phenyl, $C_{1-4}$-Alkyl, $C_{1-4}$-Alkoxy, Halogen, Trifluormethyl oder Nitro substituierte Benzoyl-, Acetal- oder $C_{1-6}$-Alkylsilyl-gruppe bedeuten,

mit einem Phosphoran der allgemeinen Formel IX

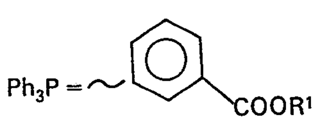

(IX)

worin
$R^1$ die oben angegebene Bedeutung hat und
Ph Phenyl ist,

umsetzt, die erhaltenen $PGF_{2\alpha}$-Derivate der allgemeinen Formel IV

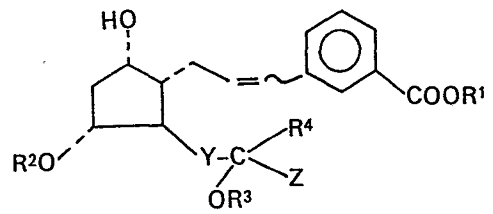

(IV)

worin
$R^1$, $R^2$, $R^3$, $R^4$, Y und Z die oben angegebene Bedeutung haben, mit einem elektrophilen Reagens der allgemeinen Formel

E–L

worin
L Halogen und
E Halogen, eine Phenylselenyl-, Sulfenyl-, Phenylsulfenyl-, N-Succinimid- oder 2,5-Dioxopyrrolidin-1-yl-gruppe oder
E–L N-Halogenhydantoin oder Pyridiniumbromid-perbromid bedeuten,

umsetzt und das erhaltene, in 5-Stellung substituierte $PGI_1$-Derivat der allgemeinen Formel V

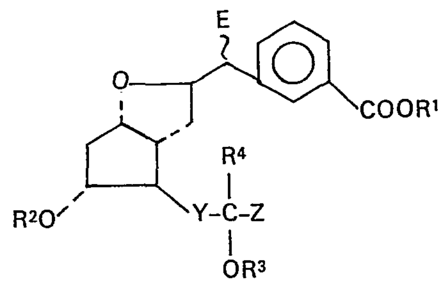

(V)

worin
$R^1$, $R^2$, $R^3$, $R^4$, Y, Z und E die oben angegebene Bedeutung haben, einer Eliminierungsreaktion unterwirft, wobei bei Verbindungen der allgemeinen Formeln IV oder V, in denen Y eine $-CH=CW-$Gruppe ist, in der W Fluor, Chlor oder Brom bedeutet, die Dehydrohalogenierung dieser Gruppe gewünschtenfalls bei dem $PGF_{2\alpha}$-Derivat der allgemeinen Formel IV vor der Umsetzung mit dem elektrophilen Reagens E–L oder bei dem $PGI_1$-Derivat der allgemeinen Formel V vor der Eliminierungsreaktion durchgeführt werden kann, und die so erhaltenen Verbindungen gegebenenfalls durch Halogenwasserstoff-Eliminierung, Verseifung, Abspalten von Schutzgruppen, Salzbildung oder Veresterung in Verbindungen der allgemeinen Formel I überführt.

Die in dem Verfahren der Erfindung als Ausgangssubstanzen verwendeten Verbindungen der allgemeinen Formel II sind entweder bekannt oder können anhand literaturbekannter Verfahren hergestellt werden (Il Farmaco Ed. Sc. 1976, 31, 763; Il Farmaco 1972, 27, 1125; J. Org. Chem. 1981, 46, 1954, 57; JACS 1974, 96, 58).

Die Phosphorane der allgemeinen Formel IX werden zweckmässig aus Triphenylbenzylphosphoniumbromid, das in der Benzylgruppe

m-substituiert ist, durch Einwirkung einer starken Base, z.B. Natriummethylsulfenylmetid (DIMSYL-NA), hergestellt. Diese Reaktion, wie auch die Umsetzung des erhaltenen Reagens werden bevorzugt in wasserfreiem Dimethylsulfoxyd ausgeführt. Die Phosphorane können in dem bei ihrer Herstellung erhaltenen Reaktionsgemisch, ohne Isolierung, in dem Verfahren der Erfindung eingesetzt werden.

Bei Einsatz von Verbindungen der allgemeinen Formel II entstehen nach der Wittig-Reaktion die $PGF_{2\alpha}$-Derivate der allgemeinen Formel IV, die nach dem Ansäuern des Reaktionsgemisches durch Extraktion isoliert werden können.

Das gebildete Produkt stellt, was die 5,6-Doppelbindung betrifft, ein Gemisch der E- und Z-Isomeren im Verhältnis 1:1 dar. Diese Isomeren können mittels Säulenchromatographie leicht voneinander getrennt werden. Die erhaltenen Verbindungen üben eine starke Stimulationswirkung auf die glatte Muskulatur aus. Überraschenderweise findet bei der Einwirkung des als starke Base zu betrachtenden Phosphorans die Eliminierung des 14-Halogenatoms und damit die Ausbildung der 13,14-Dreifachbindung nicht statt.

Gewünschtenfalls kann die Eliminierung quantitativ durchgeführt werden. Dazu verwendet man Alkoxydbasen, z.B. Kalium-tert.-butoxyd, dem der Vorzug gegeben wird, oder Natrium-tert.-butoxyd oder Natriummethoxyd, oder aber einen Überschuss an DIMSYL-Na.

Wird z.B. eine Verbindung der allgemeinen Formel VI

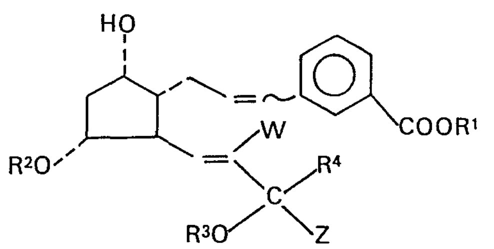

(VI)

der Eliminierungsreaktion unterworfen, so erhält man ein 13,14-Didehydro-$PGF_{2\alpha}$-Derivat der allgemeinen Formel VII

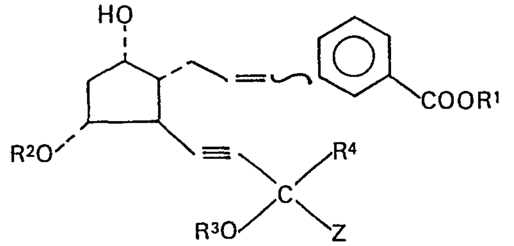

(VII)

Die Eliminierungsreaktion wird in einem polaren protischen Lösungsmittel, z.B. einem Alkohol, vorzugsweise in dem dem Alkoholat entsprechenden Alkohol, oder in aprotischen Lösungsmitteln, wie Tetrahydrofuran oder Dimethylsulfoxyd, vorgenommen. Die erhaltenen Verbindungen der allgemeinen Formel IV werden mit Hilfe von der in der Prostacyclinchemie bekannten Methoden (JACS 1977, 2006) mit einem elektrophilen Reagens der allgemeinen Formel E–L zu $PGI_1$-Derivaten der allgemeinen Formel V umgesetzt.

Als elektrophile Reagentien der allgemeinen Formel E–L werden z.B. Halogene (E = Br, L = Br bzw. E = J und L = J), wie Brom und insbesondere Jod, sowie N-Brom-succinimid oder halogenabspaltende Komplexe, wie Pyridiniumbromid-perbromid, ferner Arylselenyl-Verbindungen, wie z.B. Phenylselenylchlorid, oder Sulfenylhalogenide, Phenylsulfenylchlorid, schliesslich N-Halogenhydantoine verwendet. Für die Cyclisierungsreaktion werde als Reaktionsmedium im allgemeinen halogenierte Kohlenwasserstoffe, z.B. Methylenchlorid, Chloroform, oder aber aprotische organische Lösungsmittel, wie Tetrahydrofuran oder Toluol, verwendet. Das bei der Reaktion gebildete Rohprodukt kann ohne weitere Reinigung der Eliminierungsreaktion unterworfen werden.

Die Verbindungen der allgemeinen Formel V sind in bezug auf Stellung 6 Isomergemische der Exo- und Endoisomeren. Die Isomeren können voneinander getrennt werden, z.B. durch Säulenchromatographie. Dies ist jedoch unnötig, da aus den beiden Verbindungen der Formel V bei der Eliminierungsreaktion das $PGI_2$-Analogon der allgemeinen Formel I entsteht. Nebenreaktionen können nicht eintreten, da die Eliminierung nur in einer Richtung verläuft. Durch diese Eliminierungsreaktion wird aus den Verbindungen der allgemeinen Formel V das Endprodukt der allgemeinen Formel I erhalten.

Falls E Halogen ist, kann die Eliminierung mit einer geeigneten Base vorgenommen werden. Als Base können Alkoholate, z.B. Kalium-tert.-butoxyd oder Natriumäthylat, ferner organische Basen, wie 1,5-Diazabicyclo[3,4,0]non-5-en (DBN), 1,5-Diazabicyclo[5,4,0]undec-5-en (DBU), Pyridin, Triäthylamin, oder andere starke konjugierte Basen, z.B. Natrium-methylsulfinyl-metid, verwendet werden.

Falls E Brom oder Jod bedeutet, werden bevorzugt Kalium-tert.-butoxyd, 1,5-Diazabicyclo[3,4,0]non-5-en oder Natrium-methylsulfinylmetid verwendet.

Falls E die Phenylselenyl- oder Phenylthiogruppe bedeutet, kann die Eliminierungsreaktion auf zwei Wegen durchgeführt werden. So kann man z.B. die Ph-S- bzw. Ph-Se-Gruppe zu dem entsprechenden Sulfon bzw. Selenon oxydieren (E = $Ph\text{-}S{\lessgtr}^{O}_{O}$ oder $Ph\text{-}Se{\lessgtr}^{O}_{O}$) und dann mit den oben genannten Basen eliminieren. Man kann aber auch so vorgehen, dass man nur die Sulfoxyde bzw. Selenoxyde

$$E = \underset{\underset{O}{\|}}{Ph\text{-}S} \text{ bzw. } \underset{\underset{O}{\|}}{Ph\text{-}Se}$$

bildet und die Verbindungen der allgemeinen Formel V einer thermischen Eliminierung unterwirft.

Die Gruppen $R^1$, $R^2$ und $R^3$ der Verbindungen

der allgemeinen Formel I können nach der Eliminierung nacheinander oder gleichzeitig abgespalten oder mittels an sich bekannter chemischer Methoden in andere Gruppen $R^1$, $R^2$ und $R^3$ übergeführt werden. Erhält man z.B. bei der Eliminierung eine Verbindung, in der $R^1$ = Methyl und $R^2$ = $R^3$ = H ist, so kann man an Stelle der beiden letzteren Acylgruppen, z.B. Acetylgruppen, oder acetalartige Gruppen, z.B. Tetrahydropyranylgruppen, $C_{1-6}$-Alkyl-silylgruppen, z.B. die Dimethyl-tert.-butylsilylgruppe, mittels an sich bekannter Methoden einführen. Wird bei der Eliminierung ein Ester erhalten (z.B. $R^1$ = Methyl), der in ein Metallsalz übergeführt werden soll, so kann man den Methylester mit einer Alkalibase, z.B. Natriumhydroxyd, Kaliumhydroxyd, Lithiumhydroxyd, in wässrig-alkalischem Medium hydrolysieren und das Reaktionsgemisch anschliessend ansäuern. Die gebildete freie Säure wird durch Extraktion isoliert und später in Methanol mit der berechneten Menge Lauge zu dem gewünschten Salz umgesetzt. Die Salze der Verbindungen der allgemeinen Formel I sind kristalline Verbindungen, die gut zu handhaben sind.

Verbindungen, in denen Y eine $-CH{=}CW-$ Gruppe bedeutet, können in jedem Abschnitt des Verfahrens in der im Zusammenhang mit den Verbindungen der allgemeinen Formel IV beschriebenen Weise zu den entsprechenden Derivaten umgesetzt werden, in denen Y eine $-C{\equiv}C-$ Gruppe ist. Da aber die Eliminierung von H–W drastischere Reaktionsbedingungen als das die Eliminierung von H–E erfordert, wird die H-W-Eliminierung zweckmässig vor der H–E-Eliminierung durchgeführt.

Falls nicht anders angegeben, sind unter «$C_{1-6}$-Alkylgruppe» die Methyl-, Äthyl-, n- und Isopropyl-, n-, i-, sec.- und tert.-Butylgruppe, die unterschiedlichen Amylgruppen und Hexylgruppen, und unter $C_{1-4}$-Alkylgruppen solche mit 1–4 Kohlenstoffatomen der obigen Aufzählung zu verstehen. Die in der Beschreibung benutzte Bezeichnung $C_{1-6}$-Alkanoylgruppe betrifft Gruppen der allgemeinen Formel $C_nH_{2n+1}-CO-$, die insgesamt 1–6, vorzugsweise 1–4 Kohlenstoffatome enthalten. Beispiele hierfür sind: die Formyl-, Acetyl-, Propionyl- sowie Butyrylgruppe. Die Phenyl- und Naphthylgruppen sowie die Phenoxy- und Naphthoxygruppen können unsubstituiert oder durch Phenyl, $C_{1-4}$-Alkyl, $C_{1-4}$-Alkoxy, Halogen, Trifluormethyl oder Nitro substituiert sein. Beispiele hierfür sind die Phenyl-, Chlorphenyl-, Trifluormethylphenylgruppe sowie die entsprechenden Phenoxy- und Naphthylgruppen. Die Benzoylgruppe kann unsubstituiert oder durch Phenyl, $C_{1-4}$-Alkyl, $C_{1-4}$-Alkoxy, Halogen, Trifluormethyl oder Nitro substituiert sein. Beispiele hierfür sind: die Benezoyl- und die p-Phenylbenzoylgruppe.

Einige Beispiele für Schutzgruppen vom Acetaltyp sind die Tetrahydropyranyl-, die 1-Äthoxyäthoxy- und die 1,4-Dioxanylgruppe.

Als $C_{1-6}$-Alkyl-silylgruppen kommen vorzugsweise drei identische oder unterschiedliche $C_{1-6}$-Alkylgruppen enthaltende Trialkylsilylgruppen, z.B. bevorzugt die Dimethyl-tert.-butylsilylgruppe in Frage.

Die für $R^1$ geeigneten Kationen können Metall- oder Ammoniumkationen sein. Prinzipiell ist jedes Kation geeignet, das in der angewendeten Dosis keine oder doch keine wesentliche eigene biologische Wirkung hat, z.B. Natrium-, Kalium-, Calcium-, Lithium- oder Magnesiumion, ferner das Ammoniumkation sowie die mono-, di-, tri- und tetrasubstituierten Ammoniumionen. Die Substituenten können gegebenenfalls auch die Kristallisierbarkeit bzw. die Aufarbeitbarkeit der Salze günstig beeinflussen. Als Substituenten der Ammoniumionen kommen bevorzugt die oben genannten Alkylgruppen in Frage.

Die Erfindung betrifft auch phramzeutische Präparate, die die Verbindungen der Erfindung enthalten und in der Human- und Veterinärmedizin zur Verhütung der Aggregation der Blutplättchen, zum Auflösen bereits entstandener Thromben und zur Beeinflussung der Blutgerinnung verwendet werden können. Die Arzneimittelpräparate können neben der wirksamen Dosis einer oder mehrerer der erfindungsgemässen Verbindungen noch die für die Herstellung galenischer Zubereitungen üblichen Hilfsstoffe, wie Füll-, Träger- und Streckmittel, Stoffe zum Einstellen des pH-Wertes und des osmotischen Druckes, das Tablettieren erleichternde Zusätze und sonstige Formulierungshilfen enthalten.

Als Arzneimittelzubereitungen kommen insbesondere Tabletten, Dragees, Pillen, Pulver, Kapseln, Lösungen (Injektions- und Infusionslösungen, Tropfen, Saft), Suppositorien und Salben in Frage. Als Formulierungsformen werden besonders Tabletten, Dragees sowie Injektions- bzw. Infusionslösungen bevorzugt.

Die Dosiereinheiten der pharmazeutischen Präparate enthalten vorzugsweise jeweils eine einmalige Dosis oder einen Bruchteil davon (z.B. eine Tablette eine halbe einmalige Dosis) oder ein ganzzahliges Vielfaches davon (z.B. das Doppelte).

Da die Aktivität der erfindungsgemässen Verbindungen grössenordnungsmässig der des $PGI_2$ entspricht, können als Grundlage für die Dosierung die bekannten $PGI_2$-Dosen dienen. Eine Erhöhung der Dosen ist schon allein deswegen möglich, weil die erfindungsgemässen Verbindungen die schädliche blutdrucksenkende Nebenwirkung des $PGI_2$ nur zum $1/100$–$1/200$ Teil zeigen.

Die Erfindung wird anhand der folgenden Beispiele näher erläutert, ist jedoch nicht auf die Beispiele beschränkt.

Beispiel 1

2,3,4-Trinor-1,5-inter-m-phenylen-14-brom-20-methyl-$PGF_{2\alpha}$-methylester. In einem 100-ml-Rundkolben werden unter Stickstoffatmosphäre 722 mg (30,2 mM) Natriumhydrid und 35 ml wasserfreies Dimethylsulfoxyd vorgelegt. Die Suspension wird zuerst 30 Minuten bei 65–70°C, dann 30 Minuten bei 70–75°C gerührt. Für die Ableitung des entstehenden Wasserstoffes muss, zum Beispiel durch Verwendung eines mit Quecksilber

verschlossenen Blasenrohres, Sorge getragen werden. Zu der erhaltenen Lösung gibt man unter Stickstoffatmosphäre 7,2 g (15,1 mM) Triphenylm-carboxybenzyl-phosphoniumbromid, rührt die erhaltene Suspension bei 35°C 30 Minuten, gibt die so erhaltene rote, viskose Lösung des Phosphorans bei Raumtemperatur innerhalb von 10 Minuten zu der Lösung von 1,37 g (3,77 mM) 3,3aβ,4,5,6,6aβ-Hexahydro-2-hydroxy-4β-[2-brom-3(S)-hydroxy-1-nonenyl]-5α-hydroxy-2H-cyclopenta[b]furan in 1 ml wasserfreiem Tetrahydrofuran und rührt das Reaktionsgemisch bei 40°C 20 Minuten.

Dann wird das Reaktionsgemisch in das Gemisch von 100 ml Wasser und 30 g Eis gegossen, der pH-Wert mit Schwefelsäure auf 3–4 eingestellt und die Lösung 4× mit je 30 ml Äthylacetat extrahiert. Die vereinigten organischen Phasen werden 3× mit je 15 ml 1n Natronlauge extrahiert. Der pH-Wert der vereinigten alkalischen Extrakte wird mit 1n Schwefelsäure auf einen Wert zwischen 3,5 und 4 eingestellt, die erhaltene Lösung zunächst mit 80 ml Diäthyläther, und anschliessend mit 10 ml einmolarer ätherischer Diazomethanlösung versetzt. Die ätherische Phase wird abgetrennt, mit gesättigter Kochsalzlösung gewaschen, getrocknet und dann eingedampft, wobei man 1,78 g Rohprodukt erhält, das an einer Silikagel-Säule chromatographisch (Elutionsmittel: Äthylacetat:Benzol = 3:1) gereinigt wird. Die Fraktionen mit einem $R_f$-Wert von 0,31 (Merck Silikagel Art. 5715, Elutionsmittel wie oben) werden gesammelt und eingedampft. Man erhält 929 mg (50%) der Titelverbindung. Dünnschichtchromatographie wie oben: $R_f = 0,31$

$^1$H-NMR ($CDCl_3$): $\delta$ = 7,3–8,1 (4H), 6,2–6,7 (trans-H-5, H-6 olefin. Protonen), 5,7–5,95 (cis-H-5, H-6, H-13 olefin. Protonen), 4,0–4,35 (3H, H-9, H-11, H-15), 3,92 (3H, Methylester-$CH_3$).

Beispiel 2

2,3,4-Trinor-1,5-inter-m-phenylen-13,14-didehydro-20-methyl-$PGF_{2\alpha}$-methylester.

532 mg (1,08 mM) 2,3,4-Trinor-1,5-inter-m-phenylen-14-brom-20-methyl-$PGF_{2\alpha}$-methylester löst man in 12 ml trockenem Dimethylsulfoxyd, versetzt die Lösung mit 1,22 g (10,8 mM) Kalium-tert.-butoxyd, rührt das Gemisch 5 Minuten und verdünnt es dann mit 20 ml gesättigter Kochsalzlösung, 20 ml Wasser und 80 ml Äthylacetat. Die wässrige Phase wird mit 1n Natriumhydrogensulfatlösung auf pH-Wert = 3 angesäuert. Nach Abtrennen der organischen Phase extrahiert man die wässrige Phase 2× mit je 10 ml Äthylacetat, vereinigt die organischen Phasen, kühlt sie auf 0°C und versetzt sie mit 10–20 ml einmolarer ätherischer Diazomethanlösung. Die organische Phase wird über wasserfreiem Natriumsulfat getrocknet und nach dem Filtrieren unter vermindertem Druck eingedampft. Man erhält 474 mg Rohprodukt, das in der in Beispiel 1 beschriebenen Weise säulenchromatographisch gereinigt wird. Die Fraktionen mit dem $R_f$-Wert 0,35 werden gesammelt und unter vermindertem Druck vom Lösungsmittel befreit, wobei man 247 mg (59,8%) der Titelverbindung erhält.

Dünnschichtchromatographie: $R_f = 0,35$ (Äthylacetat:Benzol = 3:1)

$^1$H-NMR ($CDCl_3$): $\delta$ = 7,25–8,1 (4H, aromat. Protonen), 6,3–6,58 (2H, H-5, H-6), 4,0–4,5 (3H, H-9, H-11, H-15), 3,95 (s, 3H, Ester-$CH_3$).

Beispiel 3

2,3,4-Trinor-1,5-inter-m-phenylen-5-jod-14-brom-20-methyl-$PGI_1$-methylester.

577 mg (1,17 mM) 2,3,4-Trinor-1,5-inter-m-phenylen-14-brom-methyl-$PGF_{2\alpha}$-methylester löst man in 2 ml Methylenchlorid, gibt unter Rühren 11,6 ml (11,6 mM) einer Natriumhydrogencarbonatlösung der Konzentration 1 mM/ml zu und versetzt das erhaltene zweiphasige Gemisch mit 23,4 ml (2,34 mM) einer mit Methylenchlorid bereiteten Jodlösung der Konzentration 0,1 mM/ml. Das Reaktionsgemisch wird bei Raumtemperatur eine Stunde intensiv gerührt und dann mit 100 ml Äthylacetat verdünnt. Das überschüssige Jod wird durch 5%ige Natriumthiosulfatlösung entfernt, danach die organische Phase abgetrennt und die wässrige Phase 2× mit je 15 ml Äthylacetat extrahiert. Die organischen Phasen werden vereinigt, mit gesättigter Kochsalzlösung gewaschen, über wasserfreiem Natriumsulfat getrocknet, filtriert und das erhaltene Filtrat unter vermindertem Druck vom Lösungsmittel befreit. Man erhält 720 mg der Titelverbindung.

Dünnschichtchromatographie: $R_f$ = 0,6 und 0,65 [Äthylacetat und Benzol 3:1 (zweimaliges Laufenlassen)]

Beispiel 4

2,3,4-Trinor-1,5-inter-m-phenylen-5-jod-13,14-didehydro-20-methyl-$PGI_1$-methylester.

Man arbeitet in der in Beispiel 3 beschriebenen Weise, jedoch mit dem Unterschied, dass als Ausgangsverbindung 483 mg (1,17 mM) 2,3,4-Trinor-1,5-inter-m-phenylen-13,14-didehydro-20-methyl-$PGF_{2\alpha}$-methylester verwendet werden. Man erhält 603 mg der Titelverbindung.

Dünnschichtchromatographie: $R_f$ = 0,67 und 0,60 (Äthylacetat und Benzol 3:1).

Beispiel 5

2,3,4-Trinor-1,5-inter-m-phenylen-14-brom-20-methyl-$PGI_2$-methylester.

689 mg (1,11 mM) 2,3,4-Trinor-1,5-inter-m-phenylen-5-jod-14-brom-20-methyl-$PGI_1$-methylester werden in einem Rundkolben von 10 ml Volumen vorgelegt. Der Luftraum des Kolbens wird mit Stickstoff mehrmals ausgespült. Unter Inertgasatmosphäre gibt man 2 ml 1,5-Diaza-bicyclo-[4,3,0]non-5-en zu, rührt das Reaktionsgemisch bei 40°C 2 Stunden, kühlt es dann auf Raumtemperatur ab, verdünnt es mit 50 ml Äther, wäscht die ätherische Phase 3× mit je 5 ml Wasser, trocknet über wasserfreiem Natriumsulfat und dampft nach dem Filtrieren unter vermindertem Druck ein. Man erhält 543 mg Rohprodukt, das säulenchromatographisch an Silikagel mit einem

im Verhältnis 5:1 bereiteten Gemisch aus Dichlormethan und Aceton gereinigt wird. Die Fraktionen mit den $R_f$-Werten 0,73 und 0,69 werden gesammelt und getrennt voneinander eingedampft. Aus der Fraktion mit $R_f$ = 0,73 werden 216 mg (39,5%) Produkt erhalten, bei dem die 5,6-Doppelbindung (Z)-Stellung aufweist, während aus der Fraktion mit $R_f$ = 0,69 206 mg Produkt mit (E)-Geometrie isoliert werden.

Dünnschichtchromatographie: $R_f$ = 0,73 und 0,69 (Silikagelplatte von Merck, Art. 5715; Dichlormethan und Aceton 3:1; zweimaliges Laufenlassen).

$^1H$-NMR ($CDCl_3$): δ = 7,3–8,2 (m, 4H, arom. Protonen) 5,85 (d, 1H, H-19), 5,3 (s, 1H, H-5), 4,83 (m, 1H, H-9), 3,95–4,2 (m, 2H, H-11, H-15), 3,94 (s, 3H, Ester-$CH_3$).

Beispiel 6

2,3,4-Trinor-1,5-inter-m-phenylen-13,14-didehydro-20-methyl-$PGI_2$-methylester.

Man arbeitet in der in Beispiel 5 beschriebenen Weise, jedoch mit dem Unterschied, dass man als Ausgangsverbindung 599 mg (1,11 mM) 2,3,4-Trinor-1,5-inter-m-phenylen-5-jod-13,14-didehydro-20-methyl-$PGI_1$-methylester verwendet. Man erhält 218 g (52,8%) der Titelverbindung. Dünnschichtchromatographie: $R_f$ = 0,50 (Benzol und Äthylacetat 3:1).

$^1H$-NMR ($CDCl_3$): δ = 7,3–8,1 (m, 4H, arom. Protonen), 5,95 und 5,3 (s, 1H, E- bzw. Z-H-5), 4,05–4,5 (2H, H-11 und H-15), 4,75 (dt, 1H, H-9), 4,95 (3H, Ester-$CH_3$).

Beispiel 7

2,3,4-Trinor-1,5-inter-m-phenylen-13,14-didehydro-20-methyl-$PGI_2$-methylester.

494 mg (1 mM) 2,3,4-Trinor-1,5-inter-m-phenylen-14-brom-20-methyl-$PGI_2$-methylester löst man in 5 ml trockenem Dimethylsulfoxyd, fügt der Lösung bei Raumtemperatur 550 mg (5 mM) Kalium-tert.-butoxyd zu, rührt die erhaltene Suspension 5–10 Minuten, verdünnt dann mit 50 ml Wasser und stellt den pH-Wert der wässrigen Lösung mit 1n Oxalsäure auf 4 ein. Dann wird mit 30 ml, danach 2× mit je 20 ml Äthylacetat der Temperatur 0°C extrahiert. Die organischen Phasen werden vereinigt, über wasserfreiem Natriumsulfat getrocknet und nach dem Filtrieren mit 10 ml ätherischer Diazomethanlösung der Konzentration 1 M/l versetzt. Das Lösungsmittel destilliert man unter vermindertem Druck ab und reinigt das Rohprodukt chromatographisch an einer Silikagelsäule (Dichlormethan und Aceton 5:1). Die Fraktionen mit einem $R_f$-Wert von 0,5 (Benzol und Äthylacetat = 3:1) werden gesammelt und unter vermindertem Druck eingedampft. Man erhält so 255,6 mg (62%) der Titelverbindung. Die physikalischen Konstanten stimmen mit denen der gemäss Beispiel 6 erhaltenen Verbindung überein.

Wenn man als Ausgangsmaterial das gemäss Beispiel 5 hergestellte 5,6-(Z)-Produkt verwendet, so erhält man das (Z)-Isomere der Titelverbindung. Entsprechend wird, wenn man von dem gemäss Beispiel 5 hergestellten 5,6-(E)-Produkt ausgeht, das (E)-Isomere erhalten.

Beispiel 8

2,3,4-Trinor-1,5-inter-m-phenylen-14-brom-20-methyl-$PGI_2$-natriumsalz.

988 mg (2 mM) 2,3,4-Trinor-1,5-inter-m-phenylen-14-brom-20-methyl-$PGI_2$-methylester löst man in 1 ml Methanol, gibt zu der Lösung bei Raumtemperatur 1 ml (10 mM) 5n wässrige Natronlauge, rührt das Reaktionsgemisch eine Stunde bei Raumtemperatur, destilliert dann das Methanol unter vermindertem Druck ab und verdünnt den Rückstand mit einem Gemisch aus 5 ml gesättigter Kochsalzlösung und 5 ml Wasser. Die Lösung extrahiert man 3× mit je 5 ml Äther. Die wässrige Phase kühlt man auf 0°C, gibt 20 ml Äthylacetat zu und stellt sie unter intensivem Rühren auf einen pH-Wert von 4–4,5 ein. Dann trennt man die Phasen voneinander und extrahiert die wässrige Phase 3× mit je 5 ml Äthylacetat. Die organischen Phasen werden vereinigt, über wasserfreiem Natriumsulfat getrocknet und nach dem Filtrieren eingedampft, wobei 841 mg rohe Säure erhalten werden, die in 0,5 ml Methanol gelöst wird. Die erhaltene Lösung versetzt man mit 1,75 ml 1n methanolischer Natronlauge, dampft das Methanol unter vermindertem Druck ab, suspendiert das Rohprodukt in 1 ml trockenem Tetrahydrofuran und filtriert es ab. Man erhält 878,5 mg (87,5%) des im Titel genannten Salzes.

Dünnschichtchromatographie: $R_f$ = 0,54 (Merck Silikagelplatte, Art. 5715, Benzol:Dioxan:Essigsäure = 20:20:1).

Beispiel 9

2,3,4-Trinor-1,5-inter-m-phenylen-13,14-didehydro-20-methyl-$PGI_2$-natriumsalz.

Man arbeitet in der in Beispiel 8 beschriebenen Weise, jedoch mit dem Unterschied, dass man als Ausgangsverbindung 824 mg (2 mM) 2,3,4-Trinor-1,5-inter-m-phenylen-13,14-didehydro-20-methyl-$PGI_2$-methylester verwendet. Es werden 691,6 mg (82,3%) des im Titel genannten Salzes erhalten.

Dünnschichtchromatographie: $R_f$ = 0,53 (Merck Silikagelplatte, Art. 5719, Benzol:Dioxan:Essigsäure = 20:10:1).

In der in den Beispielen 1 bis 9 angegebenen Weise sind aus den entsprechenden Ausgangsverbindungen folgende Verbindungen hergestellt worden (falls nicht anders angegeben, sind die $R_f$-Werte an Merck Kieselgel Art. 5719 mit einem Gemisch aus Benzol, Dioxan und Essigsäure 20:10:1 bestimmt worden).

2,3,4-Trinor-1,5-inter-m-phenylen-13,14-didehydro-20-äthyl-$PGI_2$-natriumsalz, $R_f$ = 0,57;

2,3,4,17,18,19,20-Heptanor-1,5-inter-m-phenylen-13,14-didehydro-16-phenoxy-$PGI_2$-natriumsalz, $R_f$ = 0,6;

2,3,4,17,18,19,20-Heptanor-1,5-inter-m-phenylen-13,14-didehydro-16-(3-trifluormethyl-phenoxy)-$PGI_2$-natriumsalz, $R_f = 0{,}62$;

2,3,4-Trinor-1,5-inter-m-phenylen-14-brom-20-äthyl-$PGI_2$-natriumsalz, $R_f = 0{,}57$;

2,3,4,17,18,19,20-Heptanor-1,5-inter-m-phenylen-16-phenyl-13,14-didehydro-$PGI_2$-natriumsalz, $R_f = 0{,}69$;

2,3,4-Trinor-1,5-inter-m-phenylen-11,15-bis-(tetrahydropyran-2-yl-oxy)-13,14-didehydro-20-methyl-$PGI_2$-methylester, $R_f = 0{,}87$ (in Benzol und Äthylacetat 3:1);

2,3,4-Trinor-1,5-inter-m-phenylen-11,15-bis-(dimethyl-tert.-butyl-silyloxy)-13,14-didehydro-20-methyl-$PGI_2$-methylester, $R_f = 0{,}95$ (in Benzol und Äthylacetat 3:1).

**Patentansprüche**

1. inter-m-Phenylen-$PGI_2$-Derivate der allgemeinen Formel I

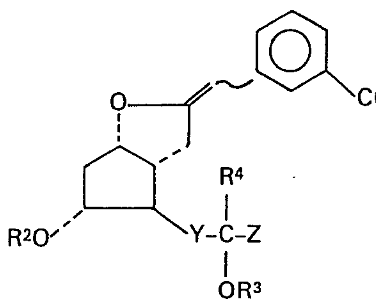

(I)

worin

$R^1$ Wasserstoff, eine $C_{1-4}$-Alkylgruppe, ein physiologisch verträgliches primäres, sekundäres, tertiäres oder quaternäres Ammoniumkation oder ein Metallkation,

$R^2$ und $R^3$ Wasserstoff,

$R^4$ Wasserstoff oder eine $C_{1-4}$-Alkylgruppe,

Y $-C{\equiv}C-$ oder die Gruppierung trans-CH=CW- bedeuten, in der W = Chlor, Brom oder Fluor ist und

Z eine gegebenenfalls durch $C_{1-4}$-Alkyl oder Fluor ein- oder mehrfach substituierte $C_{6-9}$-Alkylgruppe oder gegebenenfalls durch Phenyl, $C_{1-4}$-Alkyl, $C_{1-4}$-Alkoxy, Halogen, Trifluormethyl oder Nitro substituierte Phenylmethyl- oder Naphthylmethylgruppe oder gegebenenfalls durch Phenyl, $C_{1-4}$-Alkyl, $C_{1-4}$-Alkoxy, Halogen, Trifluormethyl oder Nitro substituierte Phenoxymethyl- oder Naphthoxymethylgruppe bedeutet,

2. 2,3,4-Trinor-1,5-inter-m-phenylen-13,14-didehydro-20-methyl-$PGI_2$-methylester.

3. 2,3,4-Trinor-1,5-inter-m-phenylen-13,14-didehydro-20-methyl-$PGI_2$-natriumsalz.

4. 2,3,4-Trinor-1,5-inter-m-phenylen-13,14-didehydro-20-äthyl-$PGI_2$-natriumsalz.

5. 2,3,4,17,18,19,20-Heptanor-1,5-inter-m-phenylen-13,14-didehydro-16-phenoxy-$PGI_2$-natriumsalz.

6. 2,3,4,17,18,19,20-Heptanor-1,5-inter-m-phenylen-13,14-didehydro-16-(3-trifluormethyl-phenoxy)-$PGI_2$-natriumsalz.

7. 2,3,4,17,18,19,20-Heptanor-1,5-inter-m-phenylen-13,14-didehydro-16-phenyl-$PGI_2$-natriumsalz.

8. inter-m-Phenylen-$PGI_2$-Derivate der allgemeinen Formel I

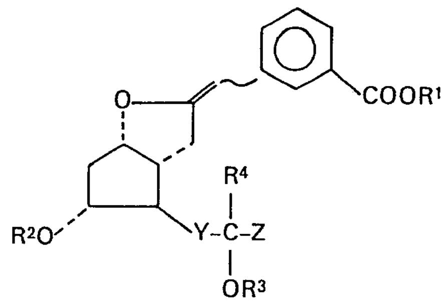

(I)

worin

$R^1$, $R^4$, Y und Z die in Anspruch 1 angegebene Bedeutung haben und

$R^2$ und $R^3$ unabhängig voneinander eine $C_{1-6}$-Alkanoyl-, gegebenenfalls durch Phenyl, $C_{1-4}$-Alkyl, $C_{1-4}$-Alkoxy, Halogen, Trifluormethyl oder Nitro substituierte Benzoyl-, Acetal- oder $C_{1-6}$-Alkylsilyl-gruppe bedeuten.

9. Verfahren zur Herstellung von inter-m-Phenylen-$PGI_2$-Derivaten der allgemeinen Formel I

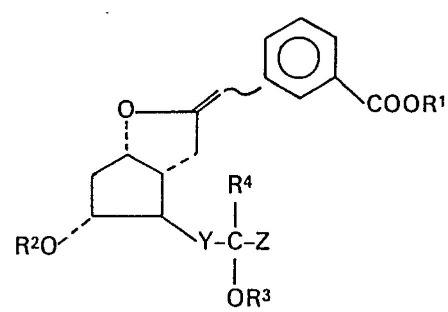

(I)

worin

$R^1$ Wasserstoff, eine $C_{1-4}$-Alkylgruppe, ein physiologisch verträgliches primäres, sekundäres, tertiäres oder quaternäres Ammoniumkation oder ein Metallkation,

$R^2$ und $R^3$ Wasserstoff,

$R^4$ Wasserstoff oder eine $C_{1-4}$-Alkylgruppe,

Y $-C{\equiv}C-$ oder die Gruppierung trans-CH=CW- bedeuten, in der W = Chlor, Brom oder Fluor ist und

Z eine gegebenenfalls durch $C_{1-4}$-Alkyl oder Fluor ein- oder mehrfach substituierte $C_{6-9}$Alkylgruppe oder gegebenenfalls durch Phenyl, $C_{1-4}$-Alkyl, $C_{1-4}$-Alkoxy, Halogen, Trifluormethyl oder Nitro substituierte Phenylmethyl- oder Naphthylmethylgruppe oder gegebenenfalls durch Phenyl, $C_{1-4}$-Alkyl, $C_{1-4}$-Alkoxy, Halogen, Trifluormethyl oder Nitro substituierte Phenoxymethyl- oder Naphthoxymethylgruppe bedeutet,

dadurch gekennzeichnet, dass man Lactole der allgemeinen Formel II

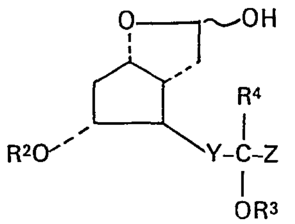
(II)

worin
$R^4$, Y und Z die oben angegebene Bedeutung haben und
$R^2$ und $R^3$ unabhängig voneinander Wasserstoff, eine $C_{1-6}$-Alkanoyl-, gegebenenfalls durch Phenyl, $C_{1-4}$-Alkyl, $C_{1-4}$-Alkoxy, Halogen, Trifluormethyl oder Nitro substituierte Benzoyl-, Acetal- oder $C_{1-6}$-Alkylsilyl-gruppe bedeuten,

mit einem Phosphoran der allgemeinen Formel IX

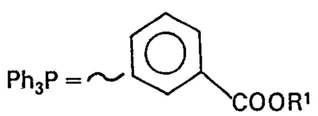
(IX)

worin
$R^1$ die oben angegebene Bedeutung hat und
Ph Phenyl ist,

umsetzt, die erhaltenen $PGF_{2\alpha}$-Derivate der allgemeinen Formel IV

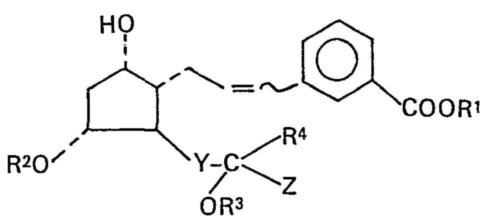
(IV)

worin
$R^1$, $R^2$, $R^3$, $R^4$, Y und Z die oben angegebene Bedeutung haben,

mit einem elektrophilen Reagens der allgemeinen Formel

E–L,

worin
L Halogen und
E Halogen, eine Phenylselenyl-, Sulfenyl-, Phenylsulfenyl-, N-Succinimid- oder 2,5-Dioxopyrrolidin-1-yl-gruppe oder
E–L N-Halogenhydantoin oder Pyridiniumbromid–perbromid bedeuten, umsetzt und das erhaltene, in 5-Stellung substituierte $PGI_1$-Derivat der allgemeinen Formel V

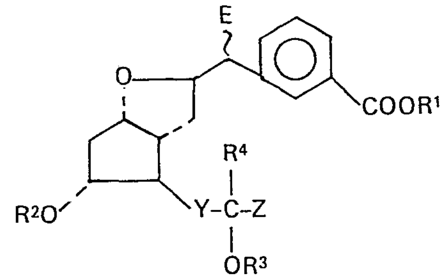
(V)

worin
$R^1$, $R^2$, $R^3$, $R^4$, Y, Z und E die oben angegebene Bedeutung haben, einer Eliminierungsreaktion unterwirft, wobei bei Verbindungen der allgemeinen Formeln IV oder V in denen Y eine $-CH{=}CW$-Gruppe ist, in der W Fluor, Chlor oder Brom bedeutet, die Dehydrohalogenierung dieser Gruppe gewünschtenfalls bei dem $PGF_{2\alpha}$-Derivat der allgemeinen Formel IV vor der Umsetzung mit dem elektrophilen Reagens E–L oder bei dem $PGI_1$-Derivat der allgemeinen Formel V vor der Eliminierungsreaktion durchgeführt werden kann, und die so erhaltenen Verbindungen gegebenenfalls durch Halogenwasserstoff-Eliminierung, Verseifung, Abspalten von Schutzgruppen, Salzbildung oder Veresterung in Verbindungen der allgemeinen Formel I überführt.

10. Verfahren nach Anspruch 9, dadurch gekennzeichnet, dass man die Dehydrohalogenierung der $-CH{=}CW$-Gruppe mit einem Alkalialkoxyd durchführt.

11. Verfahren nach Anspruch 9, dadurch gekennzeichnet, dass man als elektrophiles Reagens E–L elementares Halogen verwendet.

12. Pharmazeutische Präparate, die als Wirkstoff ein oder mehrere inter-m-Phenylen-$PGI_2$-Derivate der Ansprüche 1 bis 7 enthalten.

**Claims**

1. Inter-m-phenylene-$PGI_2$ derivatives of general formula I

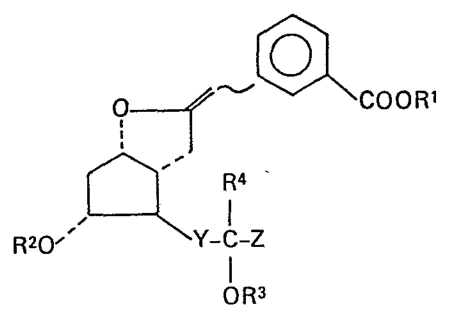
(I)

in which
$R^1$ stands for hydrogen, a $C_{1-4}$ alkyl group, a physiologically acceptable primary, secondary, tertiary or quaternary ammonium cation or a metal cation,
$R^2$ and $R^3$ stands for hydrogen,
$R^4$ stands for hydrogen or a $C_{1-4}$ alkyl group,
Y stands for –C≡C– or the grouping trans–

CH=CW–, in which W = chlorine, bromine or fluorine and

Z stands for a $C_6$–$C_9$ alkyl group optionally substituted once or several times by $C_{1-4}$ alkyl or fluorine or a phenylmethyl or naphthylmethyl group optionally substituted by phenyl, $C_{1-4}$ alkyl, $C_{1-4}$ alkoxy, halogen, trifluoromethyl or nitro, or phenoxymethyl or naphthoxymethyl group optionally substituted by phenyl, $C_{1-4}$ alkyl, $C_{1-4}$ alkoxy, halogen, trifluoromethyl or nitro.

2. 2,3,4-trinor-1,5-inter-m-phenylene-13,14-didehydro-20-methyl-$PGI_2$-methyl ester.

3. 2,3,4-trinor-1,5-inter-m-phenylene-13,14-didehydro-20-methyl-$PGI_2$-sodium salt.

4. 2,3,4-trinor-1,5-inter-m-phenylene-13,14-didehydro-20-ethyl-$PGI_2$-sodium salt.

5. 2,3,4,17,18,19,20-heptanor-1,5-inter-m-phenylene-13,14-didehydro-16-phenoxy-$PGI_2$-sodium salt.

6. 2,3,4,17,18,19,20-heptanor-1,5-inter-m-phenylene-13,14-didehydro-16-(3-trifluoro-methyl-phenoxy)-$PGI_2$-sodium salt.

7. 2,3,4,17,18,19,20-heptanor-1,5-inter-m-phenylene-13,14-didehydro-16-phenyl-$PGI_2$-sodium salt.

8. Inter-m-phenylene-$PGI_2$ derivatives of general formula I

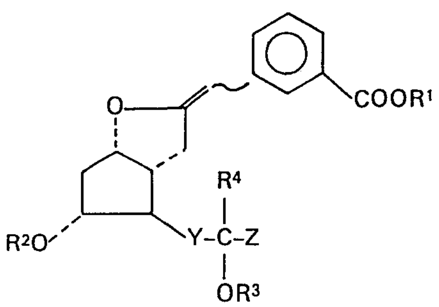

(I)

in which

$R^1$, $R^4$, Y and Z have the meanings given in claim 1 and

$R^2$ and $R^3$, independently of one another, stand for a $C_{1-6}$ alkanoyl, benzoyl (optionally substituted by phenyl, $C_{1-4}$ alkyl, $C_{1-4}$ alkoxy, halogen, trifluoromethyl or nitro), acetal or $C_{1-6}$ alkylsilyl group.

9. Process for the preparation of inter-m-phenylene-$PGI_2$-derivatives of general formula I

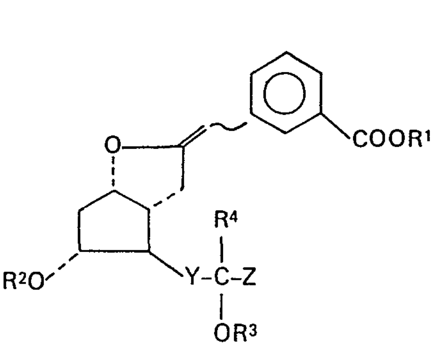

(I)

in which

$R^1$ stands for hydrogen, a $C_{1-4}$ alkyl group, a physiologically acceptable primary, secondary, tertiary or quaternary amonium cation or a metal cation,

$R^2$ and $R^3$ stands for hydrogen,

$R^4$ stands for hydrogen or a $C_{1-4}$ alkyl group,

Y stands for –C≡C– or the grouping trans-CH=CW–, in which W = chlorine, bromine or fluorine and

Z stands for a $C_6$–$C_9$ alkyl group optionally substituted once or several times by $C_{1-4}$ alkyl or fluorine or a phenylmethyl or naphthylmethyl group optionally substituted by phenyl, $C_{1-4}$ alkyl, $C_{1-4}$ alkoxy, halogen, trifluoromethyl or nitro, or phenoxymethyl or naphthoxymethyl group optionally substituted by phenyl, $C_{1-4}$ alkyl, $C_{1-4}$ alkoxy, halogen, trifluoromethyl or nitro,

characterized in that, lactols of general formula II

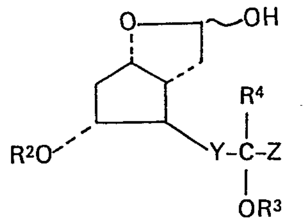

(II)

in which

$R^4$, Y and Z have the above meanings and

$R^2$ and $R^3$, independently of one another stand for hydrogen, a $C_{1-6}$ alkanoyl, a benzoyl (optionally substituted by phenyl, $C_{1-4}$ alkyl, $C_{1-4}$ alkoxy, halogen, trifluoromethyl or nitro), acetal or $C_{1-6}$ alkylsilyl group

are reacted with a phosphorane of general formula IX

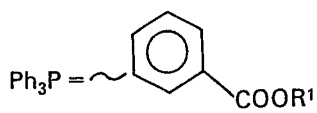

(IX)

in which

$R^1$ has the above meaning and

Ph is phenyl,

the $PGF_{2\alpha}$ derivatives obtained of general formula IV

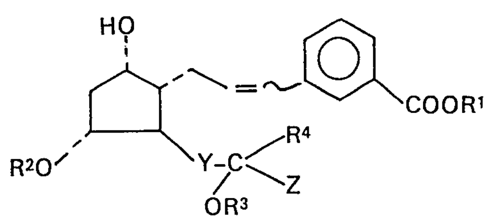

(IV)

in which

$R^1$, $R^2$, $R^3$, $R^4$, Y and Z have the above meanings are reacted with an electrophilic reagent of general formula

E–L

in which
L stands for halogen and
E stands for halogen, a phenylselenyl, sulphenyl, phenylsulphenyl, N-succinimide or 2,5-dioxopyrrolidin-1-yl group or
E–L stand for N-halogen hydantoin or pyridinium bromide-perbromide and the $PGI_1$ derivative obtained, which is substituted in the 5-position and of general formula V

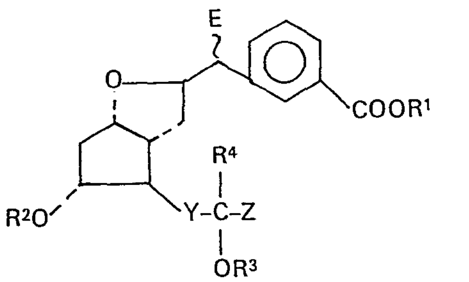

(V)

in which
$R^1$, $R^2$, $R^3$, $R^4$, Y, Z and E have the above meanings, undergoes an elimination reaction, in which in the case of compounds of general formulas IV or V, in which Y is a $-CH{=}CW$ group, in which W stands for fluorine, chlorine or bromine, the dehydrohalogenation of this group, if desired, in the case of the $PGF_{2\alpha}$ derivative of general formula IV, can be carried out prior to the reaction with the electrophilic reagent E–L or, in the case of the $PGI_1$ derivative of general formula V, prior to the elimination reaction and the thus obtained compounds can optionally be converted into the compounds of general formula I by hydrogen halide elimination, saponification, splitting off of protective groups, salt formation or esterification.

10. Process according to claim 9, characterized in that, the dehydrohalogenation of the $-CH{=}CW$ group takes place with an alkali alkoxide.

11. Process according to claim 9, characterized in that, elementary halogen is used as the electrophilic reagent E–L.

12. Pharmaceutical products containing as the active ingredient one or more inter-m-phenylene-$PGI_2$ derivatives of claims 1 to 7.

**Revendications**

1. Dérivés d'inter-m-phénylène-$PGI_2$ de formule générale I

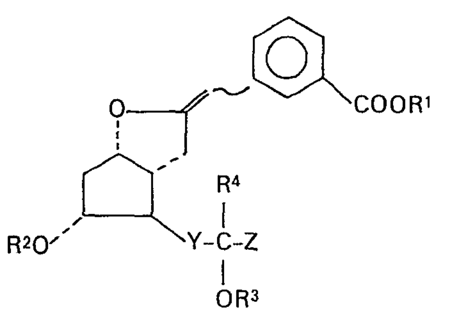

(I)

où
$R^1$ représente un hydrogène, un groupe alcoyle en $C_{1-4}$, un cation ammonium primaire, secondaire, tertiaire ou quaternaire physiologiquement acceptable ou un cation métallique,
$R^2$ et $R^3$ représentent un hydrogène,
$R^4$ représente un hydrogène ou un groupe alcoyle en $C_{1-4}$,
Y représente $-C{\equiv}C-$ ou le groupement trans-$CH{=}CW-$, où W = chlore, brome ou fluor et
Z représente un groupe alcoyle en $C_{6-9}$ éventuellement mono- ou polysubstitué par un alcoyle en $C_{1-4}$ ou un fluor ou un groupe phénylméthyle ou naphtylméthyle éventuellement mono- ou polysubstitué par phényle, alcoyle en $C_{1-4}$, alcoxy en $C_{1-4}$, halogène, trifluorométhyle ou nitro, ou un groupe phénoxyméthyle ou naphtoxyméthyle éventuellement mono- ou polysubstitué par phényle, alcoyle en $C_{1-4}$, alcoxy en $C_{1-4}$, halogène, trifluorométhyle ou nitro.

2. 2,3,4-trinor-1,5-inter-m-phénylène-13,14-didéshydro-20-méthyl-$PGI_2$-méthylester.

3. Sel de sodium de 2,3,4-trinor-1,5-inter-m-phénylène-13,14-didéshydro-20-méthyl-$PGI_2$.

4. Sel de sodium de 2,3,4-trinor-1,5-inter-m-phénylène-13,14-didéshydro-20-éthyl-$PGI_2$.

5. Sel de sodium de 2,3,4,17,18,19,20-heptanor-1,5-inter-m-phénylène-13,14-didéshydro-16-phénoxy-$PGI_2$.

6. Sel de sodium de 2,3,4,17,18,19,20-heptanor-1,5-inter-m-phénylène-13,14-didéshydro-16-(3-trifluorométhyl-phénoxy)-$PGI_2$.

7. Sel de sodium de 2,3,4,17,18,19,20-heptanor-1,5-inter-m-phénylène-13,14-didéshydro-16-phényl-$PGI_2$.

8. Dérivés d'inter-m-phénylène-$PGI_2$ de formule générale I

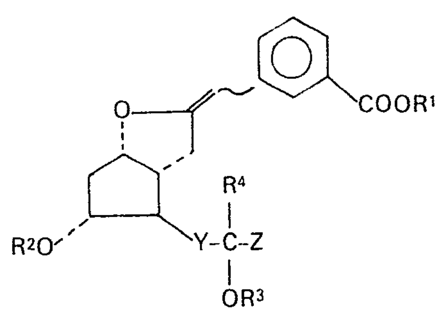

(I)

où
$R^1$, $R^4$, Y et Z ont la signification donnée dans la revendication 1 et
$R^2$ et $R^3$ représentent indépendamment l'un de l'autre un groupe alcanoyle en $C_{1-6}$, benzoyle éventuellement substitué par phényle, alcoyle en $C_{1-4}$, alcoxy en $C_{1-4}$, halogène, trifluorométhyle ou nitro, un groupe acétal ou alcoyle en $C_{1-6}$-silyle.

9. Procédé de préparation de dérivés d'inter-m-phénylène-$PGI_2$ de formule générale I

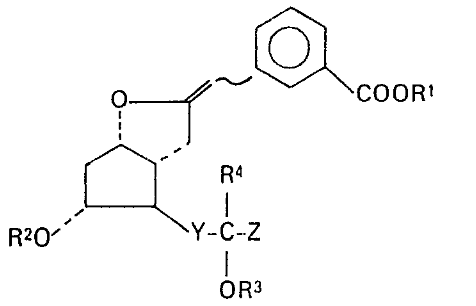

(I)

où

$R^1$ représente un hydrogène, un groupe alcoyle en $C_{1-4}$, un cation ammonium primaire, secondaire, tertiaire ou quaternaire physiologiquement acceptable ou un cation métallique,

$R^2$ et $R^3$ représentent un hydrogène,

$R^4$ représente un hydrogène ou un groupe alcoyle en $C_{1-4}$,

Y représente $-C{\equiv}C-$ ou le groupement trans-$CH{=}CW-$, où W = chlore, brome ou fluor et

Z représente un groupe alcoyle en $C_{6-9}$ éventuellement mono- ou polysubstitué par un alcoyle en $C_{1-4}$ ou un fluor ou un groupe phénylméthyle ou naphtylméthyle éventuellement mono- ou polysubstitué par phényle, alcoyle en $C_{1-4}$, alcoxy en $C_{1-4}$, halogène, trifluorométhyle ou nitro, ou un groupe phénoxyméthyle ou naphtoxyméthyle éventuellement mono- ou polysubstitué par phényle, alcoyle en $C_{1-4}$, alcoxy en $C_{1-4}$, halogène, trifluorométhyle ou nitro, caractérisé en ce qu'on fait réagir des lactols de formule générale II

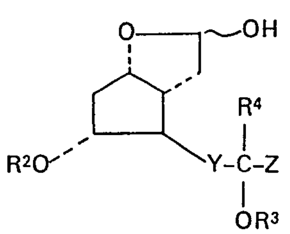

(II)

où

$R^4$, Y et Z ont la signification donnée ci-dessus et

$R^2$ et $R^3$ représentent indépendamment l'un de l'autre un hydrogène, un groupe alcanoyle en $C_{1-6}$, un groupe benzoyle éventuellement substitué par phényle, alcoyle en $C_{1-4}$, alcoxy en $C_{1-4}$, halogène, trifluorométhyle ou nitro, un groupe acétal ou alcoyle en $C_{1-6}$-silyle,

avec un phosphorane de formule générale IX

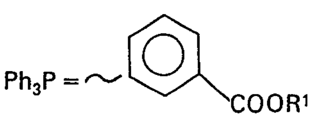

(IX)

où

$R^1$ a la signification donnée ci-dessus et

Ph est un phényle, en ce qu'on fait réagir les dérivés de $PGF_{2\alpha}$ obtenus de formule générale IV

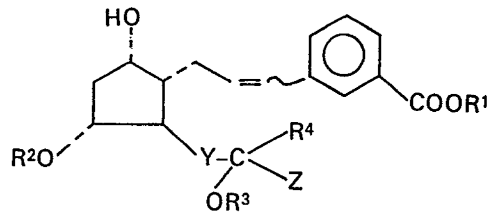

(IV)

où

$R^1$, $R^2$, $R^3$, $R^4$, Y et Z ont la signification donnée ci-dessus, avec un réactif électrophile de formule générale

E–L

où

L représente un halogène et

E représente un halogène, un groupe phénylsélényle, sulfényle, phénylsulfényle ou 2,5-dioxopyrrolidin-1-yle ou E–L représente une N-halogènehydantoïne ou le perbromure de bromure de pyridinium, et en ce qu'on soumet le dérivé de $PGI_1$ obtenu, substitué en position 5, de formule générale V

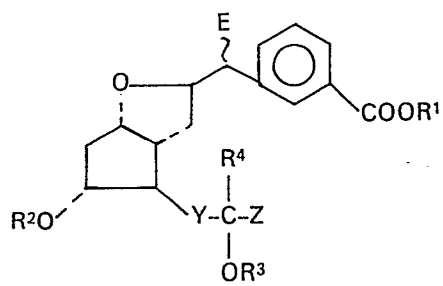

(V)

où

$R^1$, $R^2$, $R^3$, $R^4$, Y, Z et E ont la signification donnée ci-dessus, à une réaction d'élimination, où dans les composés de formules générales IV ou V où Y est un groupe $-CH{=}CW-$,

ou W représente un fluor, un chlore ou un brome, la déshydrohalogénation de ce groupe peut éventuellement s'effectuer dans le dérivé $PGF_{2\alpha}$ de formule générale IV avant la réaction avec le réactif électrophile E–L ou dans le dérivé $PGI_1$ de formule générale V avant la réaction d'élimination, et en ce qu'on transforme le cas échéant les composés ainsi obtenus par une élimination d'acide halohydrique, une saponification, une séparation de groupes protecteurs, une salification ou une estérification en composés de formule générale I.

10. Procédé selon la revendication 9, caractérisé en ce qu'on conduit la déshydrohalogénation du groupe $-CH{=}CW-$ avec un alcoxyde alcalin.

11. Procédé selon la revendication 9, caractérisé en ce qu'on utilise comme réactif électrophile E–L un halogène élémentaire.

12. Préparations pharmaceutiques qui contiennent comme substance active un ou plusieurs dérivés d'inter-m-phénylène-$PGI_2$ des revendications 1 à 7.